Europäisches Patentamt

European Patent Office

Office européen des brevets

(19)

(11) Veröffentlichungsnummer: **0 263 291 B1**

## EUROPÄISCHE PATENTSCHRIFT

(12)

(45) Veröffentlichungstag der Patentschrift: **18.03.92**

(21) Anmeldenummer: **87112390.7**

(22) Anmeldetag: **26.08.87**

(51) Int. Cl.5: **C12P 13/10**, //(C12P13/10, C12R1:13)

(54) **Verfahren zur fermentativen Herstellung von L-Arginin.**

(30) Priorität: **10.10.86 DE 3634541**

(43) Veröffentlichungstag der Anmeldung:
**13.04.88 Patentblatt 88/15**

(45) Bekanntmachung des Hinweises auf die
Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
FR-A- 2 078 850
FR-A- 2 341 648

**BIOTECHNOLOGY OF AMINO ACID PRODUC-
TION, Band 24, 1986, Kapitel 12, Seiten
131-143, Elsevier, Amsterdam, NL; H. YOSHI-
DA: "Arginine, citrulline, and ornithine"**

(73) Patentinhaber: **Degussa Aktiengesellschaft
Weissfrauenstrasse 9
W-6000 Frankfurt am Main 1(DE)**

(72) Erfinder: **Drees, Ulrich
Stapelbrede 14
W-4800 Bielefeld 1(DE)**
Erfinder: **Leuchtenberger, Wolfgang,Dr.
Geschwister-Scholl-Str. 1
W-6454 Bruchkoebel(DE)**
Erfinder: **Sahm, Hermann, Prof.
Wendelinusstr. 71
W-5171 Stetternich(DE)**

EP 0 263 291 B1

## Beschreibung

Die Erfindung betrifft die fermentative Herstellung von L-Arginin.

L-Arginin ist eine wichtige Aminosäure und seine Verwendung als Lebensmittelzusatz, Futtermittelzusatz und in medizinischen Präparationen seit langem bekannt.

Ebenso kennt man eine Reihe von Verfahren zur biotechnischen Herstellung dieser Aminosäure durch aerobes Züchten von Mikroorganismen in einem Nährmedium.

In der DE-PS 2 164 170 wird die Verwendung von Bacillus subtilis beschrieben, dessen Kultivierung in Gegenwart von Glutaminsäure die L-Argininausbeute verbessert.

Aus der GB-PS 1 229 529 ist eine Vielzahl von Bakterienstämmen bekannt, die unter Zusatz von L-Ornithin oder L-Citrullin L-Arginin in relativ geringen Mengen produzieren, so zum Beispiel Brevibacterium flavum ATCC 14 067 2,4 g/l in Gegenwart von L-Citrullin.

Eine deutlich höhere Konzentration wird gemäß US-PS 3 734 829 mit Bacillus subtilis ATCC 21 742 erzielt, wenn dem Nährmedium L-Glutaminsäure zugesetzt wurden (12,3 g/l L-Arginin).

In der DE-OS 2 108 404 wird ebenfalls ein Verfahren zur Erzeugung von L-Arginin durch Mikroorganismen beschrieben.

Mit Brevibacterium flavum ATCC 21 493 erreichte man im Fermentationsmedium eine Konzentration von 2,1 g/dl L-Arginin.

Die Wiederholung dieser Versuche unter Verwendung des von der Hinterlegungsstelle bezogenen Stammes führte allerdings zu Ausbeuten, die nur einen Bruchteil des beschriebenen Werts ausmachten.

Aufgabe der Erfindung ist ein Verfahren zur Herstellung von L-Arginin durch Mikroorganismen mit verbesserter Ausbeute.

Gegenstand der Erfindung ist ein Verfahren zur Herstellung von L-Arginin durch aerobes Züchten eines Stammes von Brevibacterium flavum in einem Nährmedium bei hierfür üblichen Temperaturen und pH-Werten und durch Isolieren des angereicherten L-Arginins aus der Fermentationsbrühe, das dadurch gekennzeichnet ist, daß man Bakterienstämme einsetzt, bei denen die N-Acetyl-L-glutaminsäure-5-phosphotransferase durch L-Arginin inhibiert wird und der Fermentationsbrühe 5 bis 50, bevorzugt 40 g/l N-Acetyl-L-glutaminsäure zusetzt.

Die Zugabe kann gleich zu Beginn oder insbesondere nach einer Wachstumsphase von 10 bis 16 h erfolgen. erfolgen.

Die N-Acetyl-L-glutaminsäure stellt zwar an sich eine Biosynthesevorstufe des L-Arginins dar. Bei dem vorliegenden Verfahren werden aber höchstens 15% ihrer Gesamtmenge verbraucht.

Der Rest kann nach Beendigung der Fermentation auf übliche Weise abgetrennt und wiederverwendet werden.

Die zur Fermentierung der erfindungsgemäß verwendeten Mikroorganismen eingesetzten Nährmedien sind herkömmlicher Natur.

Sie enthalten assimilierbare Quellen für Kohlenstoff und Stickstoff sowie anorganische Salze.

Es können geringere Mengen von organischen Nährstoffen wie Vitaminen oder Aminosäuren zugesetzt werden. Beispiele für assimilierbare Kohlenstoffquellen sind Kohlehydrate wie Glucose, Saccharose, Stärke-und Sojahydrolysate oder Melassen.

Beispiele für assimilierbare Stickstoffquellen sind organische oder anorganische Stickstoff enthaltende Verbindungen wie Nitrate, Ammoniumsalze, Ammoniakgas, Ammoniumhydroxidlösungen und Harnstoff.

Zur Erzielung guter L-Argininausbeuten wird die Fermentation aerob unter Belüftung und/oder Durchbewegung durchgeführt. Beste Ausbeuten erfordern eine pH-Kontrolle im Bereich von 5 bis 9. Der gewünschte pH-Wert kann durch Zugabe von gasförmigem oder wäßrigem Ammoniak, Calciumcarbonat, Alkalimetallhydroxid, Harnstoff, organischen oder anorganischen Säuren zu dem Medium kontrolliert werden. Ein Teil dieser Mittel kann auch dazu dienen, um assimilierbaren Stickstoff zuzuführen. Wenn die Fermentation bei 24 bis 37 °C durchgeführt wird, dann erreicht die Argininkonzentration in der Brühe innerhalb 48 bis 72 h das Maximum.

Bevorzugt eingesetzt wird als Kohlenstoffquelle Saccharose in einer Menge von 100 bis 140 g/l und als Stickstoffquelle $(NH_4)_2 SO_4$ in einer Menge von 30 bis 70 g/l, bezogen auf die Fermentationsbrühe.

Man setzt Stämme des Brevibacterium flavum ein, insbesondere den unter der Nummer ATCC 21 493 hinterlegten.

Eine Beschreibung des Biosyntheseweges zur Herstellung von L-Arginin durch Coryneform Bakterien ist enthalten in
Yoshida et al, Agric. Biol. Chem. 43 (1979) 105-111.

Beispiel 1

Brevibacterium flavum ATCC 21493 wird aus der Stammhaltung auf eine Nährbodenplatte überimpft, die 20 ml eines Mediums enthält, das folgende Zusammensetzung besitzt:

Pepton aus Fleisch    7.8 g/l
Pepton aus Casein    7.8 g/l
Hefeextrakt    2.8 g/l
Natriumchlorid    5.6 g/l

D(+)-Glucose    1.0 g/l
Agar-Agar    12.0 g/l
A. demin. ad    1.0 l
pH des gebrauchsfertigen Mediums: 7.5 ± 0.1. Das Medium kann von der Fa. Merck, Darmstadt, BRD, fertig bezogen werden.

Die beimpfte Platte wird 36 - 48 h bei 30°C im Brutschrank bebrütet. Anschließend werden 5 ml einer 0.9%igen NaCl-Lösung auf die Platte gegeben und die gewachsenen Kolonien mit Hilfe einer sterilen Impfnadel von der Oberfläche abgelöst und in Suspension gebracht. Mit 1 ml dieser Suspension wurden 25 ml eines Flüssigmediums beimpft, das mit Ausnahme von Agar-Agar mit dem obengenannten Medium identisch ist. Das Medium befindet sich in einem sterilen 500-ml-Erlenmeyerkolben mit Schikanen und Zellstoff-Stopfen.

Nach dem Animpfen wird der Kolben auf eine Inkubationsschüttelmaschine gestellt und 16 h bei 30°C und 200 Rpm inkubiert. 2.5 ml dieser Kultur werden anschließend steril aus dem Kolben entnommen und zu 25 ml eines sterilisierten Mediums gegeben, das folgende Zusammensetzung hat:

Saccharose    140 g/l
$KH_2PO_4$    1 g/l
$MgSO_4 \times 7 H_2O$    0.4 g/l
$(NH_4)_2SO_4$    50 g/l
$FeSO_4 \times 7 H_2O$    31 mg/l
$MnSO_4 \times H_2O$    8 mg/l
Sojahydrolysat    10 ml/l
Thiamin $\times$ HCl    200 ug/l
Biotin    100 ug/l
N-Acetyl-L-glutaminsäure    40 g/l
$CaCO_3$    50 g/l
pH    ad    7.2

Das Medium befindet sich in einem sterilen 500-ml-Erlenmeyerkolben mit Schikanen und Zellstoff-Stopfen. Nach Zugabe der 2.5 ml Impfkultur wird der Kolben wiederum auf eine Inkubationsschüttelmaschine gestellt und 72 h bei 30°C und 150 Rpm inkubiert. Danach wird die Biomasse durch Zentrifugation abgetrennt und der L-Arginingehalt im Medium mit Hilfe eines Aminosäureanalysators (Fa. Biotronik, München) bestimmt.

Das Medium enthält 14.7 g/l L-Arginin.

Das L-Arginin kann dann durch Ionenaustauschchromatographie und Kristallisation oder andere geeignete Verfahren aus der Lösung gewonnen werden.

Am Ende des Versuches waren noch ca. 180 mM N-Acetyl-L-glutaminsäure im Medium nachweisbar. Die Bestimmung erfolgte mittels Hochdruckflüssigkeitschromatographie.

Beispiel 2

Brevibacterium flavum ATCC 21493 wird genauso wie in Beispiel 1 behandelt, mit dem Unterschied, daß das zuletzt genannte Medium keine N-Acetyl-L-glutaminsäure enthält. Nach 72 h Inkubation und Abtrennung der Biomasse enthält das Medium ca. 4.8 g/l L-Arginin.

Beispiel 3

20 Kolben mit je 50 ml Vorkulturmedium, wie in Beispiel 1, werden mit je 1 ml Plattenabschwemmung von Brev. flavum ATCC 21493 beimpft und 16 h bei 30°C und 200 Rpm inkubiert. Nach der Inkubation werden die Kolben in einem vorher sterilisierten Gefäß vereinigt.

20 l Produktionsmedium werden in einen Fermenter mit 40 l Totalvolumen gegeben und sterilisiert. Nach der Sterilisation wird das Medium (Zusammensetzung wie in Beispiel 1) mit der Vorkultur angeimpft und 64 h bei 30°C, 800 Rpm und ca. 0,5 V/Vm Belüftung inkubiert. Der pH-Wert wird durch Zudosierung von Schwefelsäure oder Kaliumhydroxid-Lösung (ersatzweise auch Ammoniak-Lösung und gasförmigem Ammoniak) bei pH = 7,2 konstant gehalten.

Nach 64 h sind ca. 16.5 g/l L-Arginin (bezogen auf das Ausgangsvolumen) im Medium nachweisbar. Der N-Acetyl-L-glutaminsäureverbrauch während der Fermentation beträgt ca. 5 g/l.

Beispiel 4

Brevibacterium flavum ATCC 21493 wird genauso wie in Beispiel 3 behandelt, mit dem Unterschied, daß das Medium keine N-Acetyl-L-glutaminsäure enthält. Nach dem Inkubationsende beträgt der Arginingehalt im Medium ca. 4.4 g/l.

**Patentansprüche**

1. Verfahren zur fermentativen Herstellung von L-Arginin durch aerobes Züchten eines Stammes von Brevibacterium flavum in einem Nährmedium bei hierfür üblichen Temperaturen und pH-Werten und durch Isolierung des L-Arginins, dadurch gekennzeichnet,
   daß man Bakterienstämme einsetzt, bei denen die N-Acetyl-L-glutaminsäure-5-phosphotransferase durch L-Arginin inhibiert wird und der Fermentationsbrühe 5 bis 50 g/l N-Acetyl-L-glutaminsäure zusetzt.

2. Verfahren nach Anspruch 1, dadurch gekennzeichnet, daß man als Kohlenstoffquelle Saccharose einsetzt.

3. Verfahren nach den Ansprüchen 1 und 2, dadurch gekennzeichnet,
   daß man der Fermentationsbrühe $(NH_4)_2SO_4$ zusetzt.

**4.** Verfahren nach den Ansprüchen 1 bis 3, dadurch gekennzeichnet, daß man Brevibacterium flavum des Stammes ATCC 21493 einsetzt.

## Claims

**1.** A process for the fermentative preparation of L-arginine by aerobic cultivation of a strain of Brevibacterium flavum in a nutrient medium at the temperatures and pH values conventionally used for this purpose and by isolation of the L-arginine,
characterised in that bacterial strains in which L-acetyl-L-glutamic acid-5-phosphotransferase is inhibited by L-arginine are used and from 5 to 50 g/l of N-acetyl-L-glutamic acid are added to the fermentation broth.

**2.** A process according to Claim 1, characterised in that the source of carbon used is saccharose.

**3.** A process according to Claims 1 and 2, characterised in that $(NH_4)_2SO_4$ is added to the fermentation broth.

**4.** A process according to Claims 1 to 3, characterised in that Brevibacterium flavum of the strain ATCC 21493 is used.

## Revendications

**1.** Procédé de préparation fermentative d'arginine-L par culture aérobie d'une souche de brevibacterium flavum dans un milieu nutritif à des températures et des valeurs de pH habituelles dans ce but et par séparation de l'arginine-L,
caractérisé en ce qu'on utilise des souches de bactéries, avec lesquelles la N-acétyl-L-acide glutamique-5-phosphotransférose est inhibée par l'arginine-L et on ajoute au bouillon de fermentation de 5 à 50 g/l de N-acéthyl-L-acide glutamique.

**2.** Procédé selon la revendication 1, caractérisé en ce qu'on utilise de la saccharose comme source de carbone.

**3.** Procédé selon les revendications 1 et 2, caractérisé en ce qu'on ajoute au bouillon de fermentation du $(NH_4)_2 SO_4$.

**4.** Procédé selon les revendications 1 à 3, caractérisé en ce qu'on ajoute au bouillon de fermentation du brevibacterium flavum de souche ATCC 21493.